(19) Europäisches Patentamt
European Patent Office
Office européen des brevets

(11) Veröffentlichungsnummer : **0 127 816**
**B1**

(12) **EUROPÄISCHE PATENTSCHRIFT**

(45) Veröffentlichungstag der Patentschrift : **19.08.87**

(21) Anmeldenummer : **84105548.6**

(22) Anmeldetag : **16.05.84**

(51) Int. Cl.⁴ : **C 07 C121/46**, C 07 C121/00, C 07 D319/06, C 09 K 19/32, C 09 K 19/34, G 02 F 1/13

(54) **Trans-Dekalincarbonitrile.**

(30) Priorität : **01.06.83 DE 3319781**

(43) Veröffentlichungstag der Anmeldung : **12.12.84 Patentblatt 84/50**

(45) Bekanntmachung des Hinweises auf die Patenterteilung : **19.08.87 Patentblatt 87/34**

(84) Benannte Vertragsstaaten : **CH DE FR GB LI**

(56) Entgegenhaltungen :
**GB-A- 2 082 179**
**US-A- 3 925 237**
**US-A- 4 386 007**

(73) Patentinhaber : **Merck Patent Gesellschaft mit beschränkter Haftung**
**Frankfurter Strasse 250**
**D-6100 Darmstadt (DE)**

(72) Erfinder : **Eidenschink, Rudolf, Dr.**
**Kornblumenstrasse 1**
**D-6115 Münster (DE)**
Erfinder : **Weber, Georg**
**Wilhelm-Leuschner-Strasse 38**
**D-6106 Erzhausen (DE)**

**EP 0 127 816 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue trans-Dekalincarbonitrile der Formel I

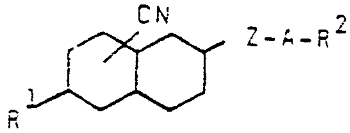

(I)

worin

$R^1$ eine Alkylgruppe mit 1-10 C-Atomen, worin bis zu zwei nicht benachbarte $CH_2$-Gruppen durch O-Atome ersetzt sein können,

Z $—CH_2CH_2—$, —CO—O—, —O—CO—, $—O—CH_2—$, $—CH_2O—$ oder eine Einfachbindung,

A 1,4-Phenylen, 1,4-Cyclohexylen, 1,3-Dioxan-2,5-diyl, 1,4-Bicyclo(2,2,2)-octylen, Pyrimidin-2,5-diyl oder eine Einfachbindung,

$R^2$ eine Alkylgruppe mit 1 bis 10 C-Atomen, worin bis zu zwei nicht benachbarte $CH_2$-Gruppen durch O-Atome ersetzt sein können, F, Cl, Br, CN, $—O—COR^3$ oder $—COOR^3$, oder, falls mindestens eine der Gruppen Z und A nicht für eine Einfachbindung steht, auch H, und

$R^3$ Alkyl mit 1 bis 6 C-Atomen

bedeuten, sowie die Säureadditionssalze der basischen unter diesen Verbindungen.

Der Einfachheit halber bedeuten im folgenden « Phe » eine 1,4-Phenylengruppe, « Cy » eine 1,4-Cyclohexylengruppe, « Dio » eine 1,3-Dioxan-2,5-diylgruppe, « Bi » eine Bicyclo-(2,2,2)-octylengruppe, « Pyr » eine Pyrimidin-2,5-diylgruppe und Dek die Gruppe

trans -

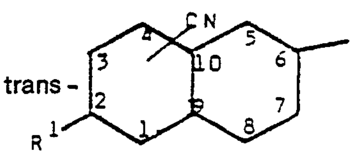

Ähnliche Verbindungen sind z. B. aus der GB-PS 2 082 179 bekannt. Die dort angegebenen Verbindungen enthalten jedoch im Gegensatz zu den vorliegenden nur zwei Substituenten am Dekalin-System.

Die Verbindungen der Formel I können wie ähnliche Verbindungen als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindungen der Formel I gelöst.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit stark negativer dielektrischer Anisotropie und damit kleiner Schwellen- bzw. Steuerspannung elektrooptischer Effekte, sehr kleiner optischer Anisotropie und vergleichsweise niedriger Viskosität herstellbar.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind ; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder daß man an eine Verbindung, die sonst der Formel I entspricht, aber im Dekalinrest keine CN-Gruppe, aber eine zusätzliche Doppelbindung enthält, HCN anlagert,

oder daß man zur Herstellung von Estern der Formel I (worin Z —CO—O— oder —O—CO— oder worin $R^2$ —O—$COR^3$ oder —$COOR^3$ bedeutet) eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von Dioxanderivaten der Formel I (worin A 1,3-Dioxan-2,5-diyl bedeutet) einen entsprechenden Aldehyd mit einem entsprechenden Diol umsetzt,
oder daß man ein entsprechendes Carbonsäureamid dehydratisiert oder ein entsprechendes Carbonsäurehalogenid mit Sulfamid umsetzt oder eine entsprechende Chlor- oder Bromverbindung mit einem Cyanid umsetzt,
oder daß man zur Herstellung von Dekalin-2- oder -6-carbonitrilen der Formel I ein Nitril der Formel II

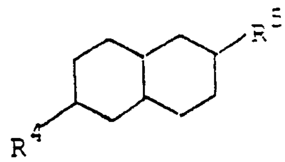

(II)

worin
(a) $R^4$ CN und $R^5$ —Z—A—$R^2$ oder
(b) $R^4$ $R^1$ und $R^5$ CN
bedeuten und
.$R^1$, A, Z, und $R^2$ die angegebenen Bedeutungen haben
mit einer Verbindung der Formel III

Q—X III

worin
Q (a) $R^1$ oder (b) $R^2$—A—Z— und
X Cl, Br, J, OH oder eine reaktionsfähig veresterte OH-Gruppe
bedeuten und
$R^1$, A, Z und $R^2$ die angegebenen Bedeutungen haben, umsetzt,
oder daß man eine Verbindung, die der Formel I entspricht, aber an Stelle einer C-C-Bindung zwischen dem die CN-Gruppe tragenden C-Atom und einem diesem benachbarten C-Atom ein zusätzliches H-Atom (an dem die CN-Gruppe tragenden C-Atom) und eine zusätzliche Gruppe X (an dem diesem benachbarten C-Atom, wobei X die angegebene Bedeutung hat) enthält, unter Abspaltung von HX cyclisiert,
oder daß man zur Herstellung von Ethern der Formel I (worin $R^1$ und/oder $R^2$ Alkylketten bedeuten worin bis zu 2 $CH_2$-Gruppen durch O-Atome ersetzt sind und/oder Z eine —$OCH_2$— oder —$CH_2O$-Gruppe ist) eine entsprechende Hydroxyverbindung verethert,
und/oder daß man gegebenenfalls eine Base der Formel I durch Behandeln mit einer Säure in eines ihrer Säureadditionssalze umwandelt,
oder daß man gegebenenfalls eine Verbindung der Formel I aus einem ihrer Säureadditionssalze durch Behandeln mit einer Base freisetzt.

Weiterhin ist Gegenstand der Erfindung die Verwendung der Verbindungen der Formel I als Komponenten flüssigkristalliner Dielektrika. Gegenstand der Erfindung sind ferner flüssigkristalline Dielektrika mit einem Gehalt an mindestens einer Verbindung der Formel I sowie elektrooptische Anzeigeelemente, die derartige Dielektrika enthalten.

Vor- und nachstehend haben A, Q, $R^1$, $R^2$, $R^3$, $R^4$, $R^5$, X und Z die angegebene Bedeutung, sofern nicht ausdrücklich etwas anderes vermerkt ist.

Die Verbindungen der Formel I umfassen dementsprechend Verbindungen der Teilformeln Ia bis Ijj :

| | |
|---|---|
| Dek—$CH_2CH_2$—Phe—$R^2$ | Ia |
| Dek—$CH_2CH_2$—Cy—$R^2$ | Ib |
| Dek—$CH_2CH_2$—Dio—$R^2$ | Ic |
| Dek—$CH_2CH_2$—Bi—$R^2$ | Id |
| Dek—$CH_2CH_2$—Pyr—$R^2$ | Ie |
| Dek—$CH_2CH_2$—$R^2$ | If |
| Dek—CO—O—Phe—$R^2$ | Ig |
| Dek—CO—O—Cy—$R^2$ | Ih |
| Dek—CO—O—Dio—$R^2$ | Ii |
| Dek—CO—O—Bi—$R^2$ | Ij |
| Dek—CO—O—Pyr—$R^2$ | Ik |
| Dek—CO—O—$R^2$ | Il |
| Dek—O—CO—Phe—$R^2$ | Im |
| Dek—O—CO—Cy—$R^2$ | In |
| Dek—O—CO—Dio—$R^2$ | Io |

| | |
|---|---|
| Dek—O—CO—Bi—$R^2$ | Ip |
| Dek—O—CO—Pyr—$R^2$ | Iq |
| Dek—O—CO—$R^2$ | Ir |
| Dek—O—$CH_2$—Phe—$R^2$ | Is |
| Dek—O—$CH_2$—Cy—$R^2$ | It |
| Dek—O—$CH_2$—Dio—$R^2$ | Iu |
| Dek—O—$CH_2$—Bi—$R^2$ | Iv |
| Dek—O—$CH_2$—Pyr—$R^2$ | Iw |
| Dek—O—$CH_2$—$R^2$ | Ix |
| Dek—$CH_2$—O—Phe—$R^2$ | Iy |
| Dek—$OH_2$—O—Cy—$R^2$ | Iz |
| Dek—$CH_2$—O—Dio—$R^2$ | Iaa |
| Dek—$CH_2$—O—Bi—$R^2$ | Ibb |
| Dek—$CH_2$—O—Pyr—$R^2$ | Icc |
| Dek—$CH_2$—O—$R^2$ | Idd |
| Dek—Phe—$R^2$ | Iee |
| Dek—Cy—$R^2$ | Iff |
| Dek—Dio—$R^2$ | Igg |
| Dek—Bi—$R^2$ | Ihh |
| Dek—Pyr—$R^2$ | Iii |
| Dek—$R^2$ | Ijj. |

(In Ijj ist $R^2$ ungleich H). Z ist bevorzugt eine Einfachbindung. A ist bevorzugt eine Einfachbindung oder Cy. Bevorzugt sind demnach Verbindungen der Formeln Iff und Ijj.

Die CN-Gruppe steht im Dekalin-System bevorzugt an einem der tertiären C-Atome, insbesondere in der 2- oder der 9-Stellung ; sie kann aber auch in 6- oder 10-Stellung, weiterhin in 1-, 3-, 4-, 5-, 7- oder 8-Stellung stehen.

In den Verbindungen der vor- und nachstehenden Formeln bedeuten $R^1$ und $R^2$ vorzugsweise Alkyl, ferner Alkoxy (insbesondere, wenn diese Reste an einer Phe-Gruppe stehen) oder eine andere Oxaalkylgruppe.

X ist vorzugsweise Cl oder Br, aber auch J, OH oder reaktionsfähig verestertes OH wie Alkylsulfonyloxy mit insbesondere 1-6 C-Atomen (z. B. Methylsulfonyloxy) oder Arylsulfonyloxy mit insbesondere 6-10 C-Atomen (z. B. Phenyl-, p-Tolyl- oder Naphthylsulfonyloxy).

In den Verbindungen der vor- und nachstehenden Formeln können die Alkylreste, in denen auch eine (« Alkoxy » bzw. « Oxaalkyl ») oder zwei (« Alkoxyalkoxy » bzw. « Dioxaalkyl ») $CH_2$-Gruppen durch O-Atome ersetzt sein können, geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2-(= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formeln I sowie Ia bis Ijj mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formeln I sowie Ia bis Iz sind diejenigen bevorzugt, in denen mindestens einer der darin enthaltenen Reste eine der angegebenen bevorzugten Bedeutungen hat und/oder worin die CN-Gruppe in einer der angegebenen bevorzugten Stellungen steht. Besonders bevorzugte kleinere Gruppen von Verbindungen sind diejenigen der Formeln Ikk und Ill :

| | |
|---|---|
| Dek-Alkyl | Ikk |
| Dek-Cy-Alkyl | Ill. |

Dabei sind diejenigen Stereoisomeren des trans-Dekalins bevorzugt, in denen die Gruppen $R^1$ und —Z—A—$R^2$ equatorial, die Cn-Gruppe axial steht.

Diejenigen der vorstehend genannten Formeln, die eine der Gruppen Dio oder Pyr enthalten, umschließen jeweils die beiden möglichen 2,5- bzw. 1,4-Stellungs-isomeren. So umschließt beispielsweise die Teilformel Igg die 2-Dek-5-$R^2$-1,3-dioxane und die 2-$R^2$-5-Dek-1,3-dioxane, die Teilformel Iii die 2-Dek-5-$R^2$-pyrimidine und die 2-$R^2$-5-Dek-pyrimidine.

Die Verbindungen der Formel I können nach an sich bekannten Methoden hergestellt werden, wie sie in der Literatur (z. B. in den Sandardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z. B. freie oder veresterte Hydroxygruppen oder aromatisch gebundene Halogenatome. Bevorzugte Ausgangsstoffe für die Reduktion entsprechen der Formel I, können aber an Stelle des Dekalinrings einen Naphthalin-, Di-, Tetra-, Hexa- oder Oktahydronaphthalinring oder einen Di-, Tetra-, Hexa-, Okta- oder Dekahydronaphthalinonring oder an Stelle eines Cyclohexanrings einen Cyclohexenring oder Cyclohexanonring und/oder an Stelle einer $—CH_2CH_2$-Gruppe eine —CH=CH-gruppe oder eine $—CH_2—CO$-Gruppe enthalten.

Die Reduktion erfolgt unter Bedingungen, bei denen die CN-Gruppe intakt bleibt, zweckmäßig durch katalytische Hydrierung bei Temperaturen zwischen etwa 0° und etwa 100° sowie Drucken zwischen etwa 1 und 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können.

Verbindungen der Formel I sind weiterhin erhältlich, indem man an ein entsprechendes Oktahydronaphthalinderivat (das sonst der Formel I entspricht, jedoch im Dekalinrest keine CN-Gruppe, dafür aber eine zusätzliche Doppelbindung enthält) Cyanwasserstoff anlagert.

Diese Anlagerung gelingt z. B. in Gegenwart eines inerten Lösungsmittels, z. B. eines halogenierten Kohlenwasserstoffs wie $CH_2Cl_2$ oder $CHCl_3$, eines Nitrils wie Acetonitril oder eines Amids wie Dimethylformamid (DMF) bei Temperaturen zwischen etwa —10 und + 150° und Drucken zwischen etwa 1 und 100 bar. Ein Zusatz von Katalysatoren kann günstig sein, z. B. kann eine HCN-Anlagerung durch Zusatz von Palladium-bis-[2,3-O-isopropyliden-2,3-dihydroxy-1,4-bis-(diphenylphosphino)-butan] katalysiert werden.

Ester der Formel I (Z = —CO—O— oder —O—CO— oder $R^2$ = $—O—COR^3$ oder $—COOR^3$, d. h. solche der Teilformeln Dek—CO—O—A—$R^2$, Dek—O—CO—A—$R^2$, Dek—Z—A—O—CO—$R^3$ oder Dek—Z—A—$COOR^3$) können auch durch Veresterung entsprechender Carbonsäuren der Formeln Dek-COOH, $R^2$—A—COOH, $R^3$—COOH oder Dek—Z—A—COOH (oder ihrer reaktionsfähigen Derivate) mit Alkoholen bzw. Phenolen der Formeln $R^2$—A—OH, Dek—OH, Dek—Z—A—OH oder $R^3$—OH (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride der Formeln Dek—CO—O—CO—$CH_3$, $R^2$—A—CO—O—CO—$CH_3$, $R^3$—CO—O—CO—$CH_3$ und Dek—Z—A—CO—O—CO—$CH_3$, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate der Formeln $R^2$—A—OM, Dek—OM, Dek—Z—A—OM und $R^3$—OM in Betracht, worin M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butyl-ether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen —50° und + 250°, vorzugsweise zwischen —20° und + 80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids

oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa — 25° und + 20°.

Dioxanderivate der Formel I (worin die Gruppe A eine 1,3-Dioxan-2,5-diyl-Gruppe bedeutet) werden zweckmäßig durch Reaktion eines entsprechenden Aldehyds der Formel Dek—Z—CHO bzw. $R^2$—CHO (oder eines seiner reaktionsfähigen Derivate) mit einem entsprechenden 1,3-Diol der Formel $R^2$—$CH(CH_2OH)_2$ bzw. Dek—Z—$CH(CH_2OH)_2$ (oder einem seiner reaktionsfähigen Derivate) hergestellt, vorzugsweise in Gegenwart eines inerten Lösungsmittels wie Benzol oder Toluol und/oder eines Katalysators, z. B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale z. B. der Formeln Dek—Z—$CH(OR^6)_2$, $R^2$—$CH(OR^6)_2$, $R^2$—$CH(CH_2O)_2$—$CHR^7$ bzw. Dek—Z—$CH(CH_2O)_2$—$CHR^7$, worin $R^6$ Alkyl mit 1-4 C-Atomen, zwei Reste $R^6$ zusammen auch Alkylen mit 2 oder 3 C-Atomen und $R^7$H, Alkyl mit 1-4 C-Atomen oder Phenyl bedeuten.

Die genannten Aldehyde und 1,3-Diole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester erhältlich.

Zur Herstellung der Nitrile der Formel I können entsprechende Säureamide, in denen an Stelle der CN-Gruppe eine $CONH_2$-Gruppe steht, dehydratisiert werden. Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $AlCl_3$ (z. B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten ; als Lösungsmittel kommen z. B. Basen wie Pyridin oder Triethylamin, aromatische Kohlenwasserstoffe wie Benzol, Toluol oder Xylol oder Amide wie DMF in Betracht.

Zur Herstellung der Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Zur Herstellung der Nitrile der Formel I kann auch eine entsprechende Chlor- oder Bromverbindung mit einem Cyanid umgesetzt werden, zweckmäßig mit einem Metallcyanid wie NaCN, KCN oder $Cu_2(CN)_2$, z. B. in Gegenwart von Pyridin in einem inerten Lösungsmittel wie DMF oder N-Methylpyrrolidon bei Temperaturen zwischen 20° und 200°.

Die bevorzugten Nitrile der Formel I, worin die Nitrilgruppe in 2- oder 6-Stellung steht, sind bevorzugt auch durch Reaktion von Nitrilen der Formel II mit Verbindungen der Formel III erhältlich. Die Nitrile der Formel II sind beispielsweise aus entsprechenden Halogeniden (entsprechend Formel II, aber Cl oder Br an Stelle von CN) und Metallcyaniden erhältlich, die Verbindungen der Formel III — soweit sie nicht bekannt sind — durch Reduktion entsprechender Carbonsäureester zu den entsprechenden Hydroxyverbindungen (III, X = OH) sowie gegebenenfalls Umsetzung derselben mit anorganischen Halogeniden wie $SOCl_2$, HBr oder HJ ; unter den Verbindungen der Formel III sind diejenigen bevorzugt, in denen Q Alkyl, $R^2$—Cy— oder $R^2$—A—$CH_2CH_2$— bedeutet. Das Nitril II wird zweckmäßig zunächst mit einer starken Base wie NaH, $NaNH_2$, Lithiumdiisopropylamid, -piperidid oder -2,5-diisopropylpiperidid oder K-tert.-Butylat in das entsprechende Carbanion übergeführt, vorzugsweise in einem inerten Lösungsmittel, beispielsweise einem Kohlenwasserstoff wie Toluol, einem Ether wie THF oder Dioxan, einem Amid wie DMF, einem Sulfoxid wie Dimethylsulfoxid oder einem Gemisch derartiger Lösungsmittel. Nach Zugabe von III (worin X von OH verschieden ist) hält man zweckmäßig 0,5 bis 16 Stunden bei Temperaturen zwischen — 30° und 100°. Eine Umsetzung von II mit III (X = OH) gelingt dagegen zweckmäßig in Gegenwart von Azodicarbonsäureestern/Triphenylphosphin in THF bei Temperaturen zwischen etwa — 30° und + 30°.

In ganz analoger Weise sind Nitrile der Formel I durch « intramolekulare Alkylierung » erhältlich, indem man ein Nitril, das der Formel I entspricht, aber an Stelle einer C-C-Bindung zwischen dem die CN-Gruppe tragenden C-Atom und einem diesem benachbarten C-Atom ein zusätzliches H-Atom (an dem die CN-Gruppe tragenden C-Atom) und eine zusätzliche Gruppe X (an dem diesem benachbarten C-Atom) enthält, unter Abspaltung von HX cyclisiert.

Als Ausgangsstoffe für diese « intramolekulare Alkylierung » kommen z. B. Verbindungen der Formeln IVa bis IV t in Betracht :

für
1-Cyan-2-$R^1$-6-(Z—A—$R^2$)-trans-dekaline :
1-Cyanmethyl-2-($CH_2CH_2$—$CHR^1$—X)-4-(Z—A—$R^2$)-cyclohexane IVa
1-X-2-($CH_2CH_2CHR^1CH_2CN$)-4-(Z—A—$R^2$)-cyclohexane IVb
für
2-Cyan-2-$R^1$-6-(Z—A—$R^2$)-trans-dekaline :
1-($CH_2X$)-2-($CH_2CH_2CHR^1CN$)-4-(Z—A—$R^2$)-cyclohexane IVc
1-($CH_2CHR^1CN$)-2-($CH_2CH_2X$)-4-(Z—A—$R^2$)-cyclohexane IVd
für
3-Cyan-2-$R^1$-6-(Z—A—$R^2$)-trans-dekaline :
1-($CH_2CHR^1$—X)-2-(2-cyanethyl)-4-(Z—A—$R^2$)-cyclohexane IVe
1-($CH_2CHR^1CH_2CN$)-2-($CH_2X$)-4-(Z—A—$R^2$)-cyclohexane IVf
für
4-Cyan-2-$R^1$-6-(Z—A—$R^2$)-trans-dekaline :
1-($CH_2CHR^1CH_2CH_2CN$)-2-X-4-(Z—A—$R^2$)-cyclohexane IVg
1-($CH_2CHR^1CH_2X$)-2-cyanmethyl-4-(Z—A—$R^2$)-cyclohexane IVh
für
5-Cyan-2-$R^1$-6-(Z—A—$R^2$)-trans-dekaline :
1-Cyanmethyl-2-($CH_2CH_2$—CHX—Z—A—$R^2$)-4-$R^1$-cyclohexane IVi
1-X-2-[$CH_2CH_2$-CH(Z—A—$R^2$)—$CH_2CN$]-4-$R^1$-cyclohexane IVj
für
6-Cyan-2-$R^1$-6-(Z—A—$R^2$)-trans-dekaline :
1-($CH_2X$)-2-[$CH_2CH_2$—CH(CN)—Z—A—$R^2$]-4-$R^1$-cyclohexane IVk
1-[$CH_2$—CH(CN)—Z—A—$R^2$]-2-($CH_2CH_2X$)-4-$R^1$-cyclohexane IVl
für
7-Cyan-2-$R^1$-6-(Z—A—$R^2$)-trans-dekaline :
1-($CH_2$—CHX—Z—A—$R^2$)-2-(2-cyanethyl)-4-$R^1$-cyclohexane IVm
1-[$CH_2$—CH(Z—A—$R^2$)—$CH_2CN$]-2-($CH_2X$)-4-$R^1$-cyclohexane IVn
für
8-Cyan-2-$R^1$-6-(Z—A—$R^2$)-trans-dekaline :
1-[$CH_2$CH(Z—A—$R^2$)—$CH_2CH_2CN$]-2-X-4-$R^1$-cyclohexane IVo
1-[$CH_2$CH(Z—A—$R^2$)—$CH_2$]-2-cyanmethyl-4-$R^1$-cyclohexane IVp
für
9-Cyan-2-$R^1$-6-(Z—A—$R^2$)-trans-dekaline :
1-[$CH_2$—CH(Z—A—$R^2$)—$CH_2CH_2X$]-2-cyan-4-$R^1$-cyclohexane IVq
1-Cyan-2-($CH_2CH_2$—$CHR^1$—$CH_2X$)-4-(Z—A—$R^2$)-cyclohexane IVr
für
10-Cyan-2-$R^1$-6-(Z—A—$R^2$)-trans-dekaline :
1-Cyan-2-[$CH_2CH_2$—CH(Z—A—$R^2$)—$CH_2X$]-4-$R^1$-cyclohexane IVs
1-($CH_2$—$CHR^1$—$CH_2CH_2X$)-2-cyan-4-cyan-4-(Z—A—$R^2$)-cyclohexane IVt.

Die Ausgangsstoffe der Formeln IVa bis IVt können beispielsweise aus den entsprechenden Carbonsäuren über die entsprechenden Chloride und Amide hergestellt werden.

Ether der Formel I (worin $R^1$ und/oder $R^2$ Alkylketten bedeuten, worin bis zu zwei $CH_2$-Gruppen durch O-Atome ersetzt sind und/oder worin Z eine —$OCH_2$— oder eine —$CH_2O$-Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z. B. durch Behandeln mit NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, DMF oder Dimethylsulfoxid oder auch einem Überschuß an wäßriger oder wäßrig-alkoholischer NaOH oder KOH bei Temperaturen zwischen etwa 20° und 100°.

Eine Base der Formel I kann mit einer Säure in das zugehörige Säureadditionssalz übergeführt werden. Für diese Umsetzung können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und-disulfonsäuren, Laurylschwefelsäure.

Umgekehrt ist es möglich, aus einem Säureadditionssalz einer Verbindung der Formel I die Base der Formel I durch Behandeln mit einer Base freizusetzen, z. B. mit einer starken anorganischen Base wie KOH oder NaOH.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Biscyclohexylbiphenyle, Phenyl- oder Cyclohexylpyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel V charakterisieren,

$$R—L—G—E—R' \qquad V$$

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | —CH=CH— | —N(O)=N— |
| | —CH=CY— | —CH=N(O)— |
| | —C≡C— | $—CH_2—CH_2—$ |
| | —CO—O— | $—CH_2—O—$ |
| | —CO—S— | $—CH_2—S—$ |
| | —CH=N— | —COO—Phe—COO— |

oder eine C—C-Einfachbindung, Y Halogen, vorzugsweise Chlor, oder —CN, und R und R' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R und R' voneinander verschieden, wobei einer dieser Reste meist eine Alkyl- oder Alkoxygruppe ist. Aber auch andere Varianten der vorgesehen Substituenten sind gebräuchlich. Viele solcher Substanzen oder auch Gemische davon sind im Handel erhältlich.

Die erfindungsgemäßen Dielektrika enthalten etwa 0,1 bis 100, vorzugsweise 10 bis 100 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z. B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent ; alle Temperaturen sind in Grad Celsius angegeben. « Übliche Aufarbeitung » bedeutet : man gibt Wasser hinzu, extrahiert mit Methylenchlorid, trennt ab, trocknet die organische Phase, dampft ein und reinigt das Produkt durch Kristallisation und/oder Chromatographie.

## Beispiel 1

Eine Lösung von 35,3 g 2-Propyl-6-cyan-6-(p-methoxybenzoylmethyl)-trans-dekalin (erhältlich aus Anisol und 2-Propyl-6-cyan-6-trans-dekalyl-acetylchlorid in Gegenwart von $AlCl_3$) in 500 ml THF wird an 5 g 10 %igem PdC bei 40° und 1 bar bis zur Aufnahme von 0,2 Mol $H_2$ hydriert. Man filtriert, dampft ein und erhält 2-Propyl-6-cyan-6-(2-p-methoxyphenylethyl)-trans-dekalin.

Analog erhält man aus 2-Cyan-2-pentyl-6-(p-acetylphenoxymethyl)-trans-dekalin das 2-Cyan-2-pentyl-6-(p-ethylphenoxymethyl)-trans-dekalin.

## Beispiel 2

In einem Autoklaven erhitzt man 28,2 g 2-Pentyl-6β-phenyl-3,4,4aα,5,6,7,8,8aβ-oktahydronaphthalin (erhältlich durch Reaktion von 6β-Phenyl-1H-(4aα,8aβ)-oktahydronaphthalin-2-on mit Pentyl-MgBr, Hydrolyse zu 2-Pentyl-6β-phenyl-3,4,4aα,5,6,7,8,8aβ-oktahydronaphthalin-2-ol und Dehydratisierung), 2,7 g flüssiges HCN, 0,1 g Palladium-bis-[2,3-O-isopropyliden-2,3-dihydroxy-1,4-bis-(diphenylphosphino)-butan] und 100 ml Acetonitril 1 Stunde auf 130°. Nach Abkühlen, Eindampfen und üblicher Aufarbeitung erhält man 2β-Cyan-2α-pentyl-6β-phenyl-trans-dekalin.

Analog sind durch Anlagerung von HCN an die entsprechenden Dekaline erhältlich :

2β-Cyan-2α-propyl-6β-(trans-4-propylcyclohexyl)-trans-dekalin
2β-Cyan-2α-propyl-6β-(trans-4-butylcyclohexyl)-trans-dekalin
2β-Cyan-2α-propyl-6β-(trans-4-pentylcyclohexyl)-trans-dekalin
2β-Cyan-2α-propyl-6β-(trans-4-hexylcyclohexyl)-trans-dekalin
2β-Cyan-2α-propyl-6β-(trans-4-heptylcyclohexyl)-trans-dekalin

## Beispiel 3

Man kocht 17 g trans-4-Propylcyclohexancarbonsäure 1 Std. mit 24 g $SOCl_2$, löst das erhaltene rohe Säurechlorid in 150 ml Toluol, versetzt mit 8 ml Pyridin und 16,7 g 2-Cyan-2-propyl-trans-dekalin-6-ol (erhältlich durch Alkylierung von 2-Cyan-trans-dekalin-6-ol) und kocht 2 Stunden. Nach Abkühlen und üblicher Aufarbeitung erhält man trans-4-Propylcyclohexancarbonsäure-(2-cyan-2-propyl-6-trans-dekalyl-ester).

Analog erhält man aus den entsprechenden Säurechloriden durch Veresterung :

2-Cyan-2-propyl-trans-dekalin-6-carbonsäure-(p-fluorphenylester)
2-Cyan-2-pentyl-trans-dekalin-6-carbonsäure-(p-cyanphenylester)
2-Methylpyrimidin-5-carbonsäure-(2-cyan-2-pentyl-6-trans-dekalylester).

## Beispiel 4

Ein Gemisch von 1,2 g 2-Proylpropan-1,3-diol, 2,33 g 2-Cyan-2-propyl-6-formyl-trans-dekalin (erhältlich durch Anlagerung von HCN an 2-Propyl-6-hydroxymethyl-3,4,4aα-5,6,7,8,8aβ-oktahydronaphthalin zu 2-Cyan-2-propyl-6-hydroxymethyl-trans-dekalin und Oxydation), 0,01 g p-Toluolsulfonsäure und 15 ml Toluol wird am Wasserabscheider 3 Std. gekocht, abgekühlt, mit Wasser gewaschen und eingedampft. Man erhält 2-(2-Cyan-2-propyl-6-trans-dekalyl)-5-propyl-1,3-dioxan.

Analog sind die homologen 2-(2-Cyan-2-alkyl-6-trans-dekalyl)-5-alkyl-1,3-dioxane erhältlich.

## Beispiel 5

Eine Lösung von 26,5 g 2,6-Dipropyl-trans-dekalin-9-carboxamid (erhältlich durch Reaktion von 6-Propyl-trans-dekalin-2-on mit Propyl-MgBr zu 2,6β-Dipropyl-trans-dekalin-2-ol und Umsetzung mit $H_2SO_4$/HCOOH zu 2,6β-Dipropyl-trans-dekalin-9-carbonsäure, Überführung in das Säurechlorid und Reaktion mit $NH_3$) in 500 ml DMF wird bei 50° unter Rühren tropfenweise mit 65 g $POCl_3$ versetzt. Nach weiterem einstündigem Rühren gießt man auf Eis, arbeitet wie üblich auf und erhält 2,6-Dipropyl-9-cyan-trans-dekalin.

Analog erhält man durch Dehydratisierung der entsprechenden Amide :

2-Propyl-6-butyl-9-cyan-trans-dekalin
2-Propyl-6-pentyl-9-cyan-trans-dekalin
2-Propyl-6-hexyl-9-cyan-trans-dekalin
2-Propyl-6-heptyl-9-cyan-trans-dekalin
2-Butyl-6-propyl-9-cyan-trans-dekalin
2,6-Dibutyl-9-cyan-trans-dekalin
2-Butyl-6-pentyl-9-cyan-trans-dekalin
2-Butyl-6-hexyl-9-cyan-trans-dekalin
2-Butyl-6-heptyl-9-cyan-trans-dekalin
2-Pentyl-6-propyl-9-cyan-trans-dekalin
2-Pentyl-6-butyl-9-cyan-trans-dekalin
2,6-Dipentyl-9-cyan-trans-dekalin
2-Pentyl-6-hexyl-9-cyan-trans-dekalin
2-Pentyl-6-heptyl-9-cyan-trans-dekalin
2-Hexyl-6-propyl-9-cyan-trans-dekalin
2-Hexyl-6-butyl-9-cyan-trans-dekalin
2-Hexyl-6-pentyl-9-cyan-trans-dekalin

2,6-Dihexyl-9-cyan-trans-dekalin
2-Hexyl-6-heptyl-9-cyan-trans-dekalin
2-Heptyl-6-propyl-9-cyan-trans-dekalin
2-Heptyl-6-butyl-9-cyan-trans-dekalin
2-Heptyl-6-pentyl-9-cyan-trans-dekalin
2-Heptyl-6-hexyl-9-cyan-trans-dekalin
2,6-Diheptyl-9-cyan-trans-dekalin.

Beispiel 6

Eine Lösung von 36,7 g 2-Propyl-6-(trans-4-propylcyclohexyl)-trans-dekalin-9-carbonylchlorid [erhältlich durch Reaktion von 6-(trans-4-Propylcyclohexyl)-trans-dekalin-2-on mit Propyl-MgBr zu 2-Propyl-6-(trans-4-propylcyclohexyl)-trans-dekalin-2-ol, Umsetzung mit $H_2SO_4$/HCOOH zu 2-Propyl-6-(trans-4-propylcyclohexyl)-trans-dekalin-9-carbonsäure und Reaktion mit $SOCl_2$] und 8 g Sulfamid in 500 ml Tetramethylensulfon wird 4 Std. auf 120° erhitzt, eingedampft und wie üblich aufgearbeitet. Man erhält 2-Propyl-6-(trans-4-propylcyclohexyl)-9-cyan-trans-dekalin.

Analog sind aus den entsprechenden Säurechloriden erhältlich :

2-Propyl-6-(trans-4-butylcyclohexyl)-9-cyan-trans-dekalin
2-Propyl-6-(trans-4-pentylcyclohexyl)-9-cyan-trans-dekalin
2-Propyl-6-(trans-4-hexylcyclohexyl)-9-cyan-trans-dekalin
2-Propyl-6-(trans-4-heptylcyclohexyl)-9-cyan-trans-dekalin
2-Butyl-6-(trans-4-propylcyclohexyl)-9-cyan-trans-dekalin
2-Butyl-6-(trans-4-butylcyclohexyl)-9-cyan-trans-dekalin
2-Butyl-6-(trans-4-pentylcyclohexyl)-9-cyan-trans-dekalin
2-Butyl-6-(trans-4-hexylcyclohexyl)-9-cyan-trans-dekalin
2-Butyl-6-(trans-4-heptylcyclohexyl)-9-cyan-trans-dekalin
2-Pentyl-6-(trans-4-propylcyclohexyl)-9-cyan-trans-dekalin
2-Pentyl-6-(trans-4-butylcyclohexyl)-9-cyan-trans-dekalin
2-Pentyl-6-(trans-4-pentylcyclohexyl)-9-cyan-trans-dekalin
2-Pentyl-6-(trans-4-hexylcyclohexyl)-9-cyan-trans-dekalin
2-Pentyl-6-(trans-4-heptylcyclohexyl)-9-cyan-trans-dekalin
2-Hexyl-6-(trans-4-propylcyclohexyl)-9-cyan-trans-dekalin
2-Hexyl-6-(trans-4-butylcyclohexyl)-9-cyan-trans-dekalin
2-Hexyl-6-(trans-4-pentylcyclohexyl)-9-cyan-trans-dekalin
2-Hexyl-6-(trans-4-hexylcyclohexyl)-9-cyan-trans-dekalin
2-Hexyl-6-(trans-4-heptylcyclohexyl)-9-cyan-trans-dekalin
2-Heptyl-6-(trans-4-propylcyclohexyl)-9-cyan-trans-dekalin
2-Heptyl-6-(trans-4-butylcyclohexyl)-9-cyan-trans-dekalin
2-Heptyl-6-(trans-4-pentylcyclohexyl)-9-cyan-trans-dekalin
2-Heptyl-6-(trans-4-hexylcyclohexyl)-9-cyan-trans-dekalin
2-Heptyl-6-(trans-4-heptylcyclohexyl)-9-cyan-trans-dekalin

Beispiel 7

Man löst 8 g KCN in wenig Wasser und tropft unter Rühren eine Lösung von 28,5 g 2-Chlor-2-propyl-6-pentyl-trans-dekalin (erhältlich durch Reaktion von 6-Pentyl-trans-dekalin-2-on mit Propyl-MgBr zu 2-Hydroxy-2-propyl-6-pentyl-trans-dekalin und Umsetzung mit HCl) in 200 ml Ethanol hinzu. Nach 3 std. Kochen dampft man ein, arbeitet wie üblich auf und erhält 2-Cyan-2-propyl-6-pentyl-trans-dekalin.

Analog erhält man aus den entsprechenden Chlor- oder Bromverbindungen.

1-Cyan-2-pentyl-6-(4-propylbicyclo(2,2,2,)octylen)-trans-dekalin
2-Methoxymethyl-4-cyan-6-p-fluorphenyl-trans-dekalin
2-Octyl-5-cyan-6-p-methoxymethoxyphenyl-trans-dekalin
2-(2,6-Dioxaheptyl)-6-(trans-4-methoxymethyl-cyclohexyl)-7-cyan-trans-dekalin.

Beispiel 8

Man löst 20,5 g 2-Cyan-6β-propyl-trans-dekalin (erhältlich durch Reaktion von 6β-Propyl-trans-dekalin-2-on mit Triphenylphosphin-methoxymethylen und $HClO_4$ zu 2-Formyl-6β-propyl-trans-dekalin, Überführung in das Oxim und Dehydratisierung) und 41 g Butylbromid in 70 ml Toluol, versetzt mit 4,3 g $NaNH_2$ (50 % in Toluol) und kocht 5 Stunden. Nach üblicher Aufarbeitung erhält man 2β-Cyan-2α-butyl-6β-propyl-trans-dekalin.

Analog erhält man durch Alkylierung der entsprechenden Nitrile :

2β-Cyan-2α-methyl-6β-propyl-trans-dekalin
2β-Cyan-2α-methyl-6β-butyl-trans-dekalin
2β-Cyan-2α-methyl-6β-pentyl-trans-dekalin
2β-Cyan-2α-methyl-6β-hexyl-trans-dekalin
2β-Cyan-2α-methyl-6β-heptyl-trans-dekalin
2β-Cyan-2α-ethyl-6β-propyl-trans-dekalin
2β-Cyan-2α-ethyl-6β-butyl-trans-dekalin
2β-Cyan-2α-ethyl-6β-pentyl-trans-dekalin
2β-Cyan-2α-ethyl-6β-hexyl-trans-dekalin
2β-Cyan-2α-ethyl-6β-heptyl-trans-dekalin
2β-Cyan-2α,6β-dipropyl-trans-dekalin
2β-Cyan-2α-propyl-6β-butyl-trans-dekalin
2β-Cyan-2α-propyl-6β-pentyl-trans-dekalin
2β-Cyan-2α-propyl-6β-hexyl-trans-dekalin
2β-Cyan-2α-propyl-6β-heptyl-trans-dekalin
2β-Cyan-2α,6β-dibutyl-trans-dekalin
2β-Cyan-2α-butyl-6β-pentyl-trans-dekalin
2β-Cyan-2α-butyl-6β-hexyl-trans-dekalin
2β-Cyan-2α-butyl-6β-heptyl-trans-dekalin
2β-Cyan-2α-pentyl-6β-propyl-trans-dekalin
2β-Cyan-2α-pentyl-6β-butyl-trans-dekalin
2β-Cyan-2α,6β-dipentyl-trans-dekalin, F. 9°, K. — 90°
2β-Cyan-2α-pentyl-6β-hexyl-trans-dekalin
2β-Cyan-2α-pentyl-6β-heptyl-trans-dekalin
2β-Cyan-2α-hexyl-6β-propyl-trans-dekalin
2β-Cyan-2α-hexyl-6β-butyl-trans-dekalin
2β-Cyan-2α-hexyl-6β-pentyl-trans-dekalin
2β-Cyan-2α,6β-dihexyl-trans-dekalin
2β-Cyan-2α-hexyl-6β-heptyl-trans-dekalin
2β-Cyan-2α-heptyl-6β-propyl-trans-dekalin
2β-Cyan-2α-heptyl-6β-butyl-trans-dekalin
2β-Cyan-2α-heptyl-6β-pentyl-trans-dekalin
2β-Cyan-2α-heptyl-6β-hexyl-trans-dekalin
2β-Cyan-2α,6β-diheptyl-trans-dekalin
2β-Cyan-2α-(2-methoxyethyl)-6β-propyl-trans-dekalin.

## Beispiel 8a

Analog Beispiel 8 erhält man aus 2-Cyan-6β-(trans-4-propylcyclohexyl)-trans-dekalin [erhältlich durch Reaktion von 4-(trans-4-Propylcyclohexyl)-cyclohexanon mit Morpholin zum Enamin, Reaktion mit Methylvinylketon zu 2-(3-Oxobutyl)-4-(trans-4-propylcyclohexyl)-cyclohexanon, Cyclisierung mit NaOH zu 6-(trans-4-Propylcyclohexyl)-2,3,4,4a,5,6,7,8-oktahydronaphthalin-2-on, Reduktion mit Li/$NH_3$ zu 6-(trans-4-Propylcyclohexyl)-dekalin-2-on, Umwandlung in das 2,4,6-Triisopropylbenzolsulfonylhydrazon und Reaktion mit KCN] und Pentylbromid das 2β-Cyan-2α-pentyl-6β-(trans-4-propylcyclohexyl)-trans-dekalin, F. 61°, K. 104°.

Analog erhält man

2β-Cyan-2α-methyl-6β-(trans-4-propylcyclohexyl)-trans-dekalin
2β-Cyan-2α-methyl-6β-(trans-4-butylcyclohexyl)-trans-dekalin
2β-Cyan-2α-methyl-6β-(trans-4-pentylcyclohexyl)-trans-dekalin
2β-Cyan-2α-methyl-6β-(trans-4-hexylcyclohexyl)-trans-dekalin
2β-Cyan-2α-methyl-6β-(trans-4-heptylcyclohexyl)-trans-dekalin
2β-Cyan-2α-ethyl-6β-(trans-4-propylcyclohexyl)-trans-dekalin
2β-Cyan-2α-ethyl-6β-(trans-4-butylcyclohexyl)-trans-dekalin
2β-Cyan-2α-ethyl-6β-(trans-4-pentylcyclohexyl)-trans-dekalin
2β-Cyan-2α-ethyl-6β-(trans-4-hexylcyclohexyl)-trans-dekalin
2β-Cyan-2α-ethyl-6β-(trans-4-heptylcyclohexyl)-trans-dekalin
2β-Cyan-2α-propyl-6β-(trans-4-propylcyclohexyl)-trans-dekalin
2β-Cyan-2α-propyl-6β-(trans-4-butylcyclohexyl)-trans-dekalin
2β-Cyan-2α-propyl-6β-(trans-4-pentylcyclohexyl)-trans-dekalin
2β-Cyan-2α-propyl-6β-(trans-4-hexylcyclohexyl)-trans-dekalin
2β-Cyan-2α-propyl-6β-(trans-4-heptylcyclohexyl)-trans-dekalin
2β-Cyan-2α-butyl-6β-(trans-4-propylcyclohexyl)-trans-dekalin
2β-Cyan-2α-butyl-6β-(trans-4-butylcyclohexyl)-trans-dekalin

2β-Cyan-2α-butyl-6β-(trans-4-pentylcyclohexyl)-trans-dekalin
2β-Cyan-2α-butyl-6β-(trans-4-hexylcyclohexyl)-trans-dekalin
2β-Cyan-2α-butyl-6β-(trans-4-heptylcyclohexyl)-trans-dekalin
2β-Cyan-2α-pentyl-6β-(trans-4-butylcyclohexyl)-trans-dekalin
2β-Cyan-2α-pentyl-6β-(trans-4-pentylcyclohexyl)-trans-dekalin
2β-Cyan-2α-pentyl-6β-(trans-4-hexylcyclohexyl)-trans-dekalin
2β-Cyan-2α-pentyl-6β-(trans-4-heptylcyclohexyl)-trans-dekalin
2β-Cyan-2α-hexyl-6β-(trans-4-propylcyclohexyl)-trans-dekalin
2β-Cyan-2α-hexyl-6β-(trans-4-butylcyclohexyl)-trans-dekalin
2β-Cyan-2α-hexyl-6β-(trans-4-pentylcyclohexyl)-trans-dekalin
2β-Cyan-2α-hexyl-6β-(trans-4-hexylcyclohexyl)-trans-dekalin
2β-Cyan-2α-hexyl-6β-(trans-4-heptylcyclohexyl)-trans-dekalin
2β-Cyan-2α-heptyl-6β-(trans-4-propylcyclohexyl)-trans-dekalin
2β-Cyan-2α-heptyl-6β-(trans-4-butylcyclohexyl)-trans-dekalin
2β-Cyan-2α-heptyl-6β-(trans-4-pentylcyclohexyl)-trans-dekalin
2β-Cyan-2α-heptyl-6β-(trans-4-hexylcyclohexyl)-trans-dekalin
2β-Cyan-2α-heptyl-6β-(trans-4-heptylcyclohexyl)-trans-dekalin

## Beispiel 9

Eine Lösung von 41,9 g 2-[2-(2-p-Toluolsulfonyloxymethyl-5-propylcyclohexyl)-ethyl]-valeronitril [erhältlich durch Reaktion von p-Propylbenzyl-2-tetrahydropyranylether mit 2-Propylbernsteinsäureanhydrid/$AlCl_3$ zu 2-(2-Tetrahydropyranylmoxymethyl-5-propyl-benzoylmethyl)-valeriansäure, Hydrierung zu 2-[2-(2-Tetrahydropyranyloxymethyl-5-propylcyclohexyl)-ethyl]-valeriansäure, aufeinanderfolgende Reaktionen mit $SOCl_2$ und $NH_3$ zu 2-[2-(2-Hydroxymethyl-5-propylcyclohexyl)-ethyl]-valeriansäureamid, Dehydratisierung mit Dicyclohexylcarbodiimid zum Nitril und Tosylierung] in 250 ml Dimethylsulfoxid wird unter Rühren mit 6 g NaH (50 %ig in Paraffin) versetzt ; dabei hält man die Temperatur unter 35°. Man rührt noch 2 Stunden, arbeitet wie üblich auf und erhält 2-Cyan-2,6-dipropyl-trans-dekalin.

Analog erhält man aus 2-(2-Bromethyl-4-methoxycyclohexylmethyl)-valeronitril [erhältlich durch Chlormethylierung von 2-(m-Methoxyphenyl)-ethanol zu 2-(2-Chlormethyl-5-methoxyphenyl)-ethanol, Reaktion mit Propylmalonsäurediethylester, Verseifung und Decarboxylierung zu 2-[2-(2-Hydroxyethyl)-4-methoxybenzyl]-valeriansäure, Hydrierung zu 2-[2-(2-Hydroxyethyl)-4-methoxycyclohexylmethyl]-valeriansäure, Überführung in das Amid, Reaktion mit $SOBr_2$ zu 2-(2-Bromethyl-4-methoxy-cyclohexylmethyl)-valeramid und Dehydratisierung] das 2-Cyan-2-propyl-6-methoxy-trans-dekalin.

## Beispiel 10

Ein Gemisch von 29,7 g 2-Cyan-2-propyl-6-p-hydroxyphenyl-trans-dekalin [erhältlich durch Reaktion von 4-p-Hydroxyphenylcyclohexanon mit Morpholin zu 1-Morpholino-4-p-hydroxyphenylcyclohexen, Umsetzung mit Methylvinylketon und Cyclisierung zu 6-p-Hydroxyphenyl-2,3,4,4a,5,6,7,8-oktahydronaphthalin-2-on, Hydrierung zu 6-p-Hydroxyphenyl-trans-dekalin-2-on, Grignard-Reaktion mit PropylMgBr und Hydrolyse zu 6-p-Hydroxyphenyl-2-propyl-dekalin-2-ol, Dehydratisierung und Anlagerung von HCN], 6,9 g $K_2CO_3$, 25 g Hexyljodid und 250 ml DMF wird unter Rühren 16 Std. auf 80° erhitzt, dann abgekühlt und wie üblich aufgearbeitet. Man erhält 2-Cyan-2-propyl-6-p-hexyloxyphenyl-trans-dekalin.

Analog sind die homologen 2-Cyan-2-alkyl-6-p-alkoxyphenyl-trans-dekaline erhältlich.

Es folgen Beispiele für erfindungsgemäße Dielektrika mit einem Gehalt an mindestens einer Verbindung der Formel I :

## Beispiel A

Man stellt ein Gemisch her aus

11 % 2β-Cyan-2α-pentyl-6β-propyl-trans-dekalin
24 % 2β-Cyan-2α-heptyl-6β-propyl-trans-dekalin
21 % 2β-Cyan-2α-propyl-6β-pentyl-trans-dekalin
21 % 2β-Cyan-2α,6β-dipentyl-trans-dekalin
13 % 2β-Cyan-2α-propyl-6β-heptyl-trans-dekalin und
10 % 4-(trans-4-Pentylcyclohexyl)-4'-(trans-4-propylcyclohexyl)-biphenyl.

## Beispiel B

In 98 Gewichtsteilen des Gemisches nach Beispiel A löst man 2 Gewichtsteile des blauen Farbstoffs 4,8-Diamino-1,5-dihydroxy-2-p-methoxyphenyl-anthrachinon.

## Beispiel C

Man stellt ein Gemisch her aus

9 % 2β-Cyan-2α-pentyl-6β-propyl-trans-dekalin
19 % 2β-Cyan-2α-heptyl-6β-propyl-trans-dekalin
17 % 2β-Cyan-2α-propyl-6β-pentyl-trans-dekalin
17 % 2β-Cyan-2α,6β-dipentyl-trans-dekalin
10 % 2β-Cyan-2α-propyl-6β-heptyl-trans-dekalin und
28 % 4-Ethyl-2'-fluor-4-(trans-4-pentylcyclohexyl)-biphenyl.

Beispiel D

In 98 Gewichtsteilen des Gemisches nach Beispiel C löst man 2 Gewichtsteile des roten Farbstoffs 1-p-Dimethylaminobenzylidenamino-4-p-cyanphenylazonaphthalin.

Beispiel E

Man stellt ein Gemisch her aus

13 % 2β-Cyan-2α-pentyl-6β-propyl-trans-dekalin
27 % 2β-Cyan-2α-heptyl-6β-propyl-trans-dekalin
23 % 2β-Cyan-2α-propyl-6β-pentyl-trans-dekalin
23 % 2β-Cyan-2α,6β-dipentyl-trans-dekalin und
14 % 2β-Cyan-1-propyl-6β-heptyl-trans-dekalin.

Beispiel F

In 99 Gewichtsteilen des Gemisches nach Beispiel E löst man 1 Gewichsteil Perylen-3,9-bis-carbonsäure-bis-(p-isopropylphenylester).

Beispiel G

Man stellt ein Gemisch her aus

12 % 2β-Cyan-2α-pentyl-6β-propyl-trans-dekalin
25 % 2β-Cyan-2α-heptyl-6β-propyl-trans-dekalin
22 % 2β-Cyan-2α-propyl-6β-pentyl-trans-dekalin
22 % 2β-Cyan-2α,6β-dipentyl-trans-dekalin
13 % 2β-Cyan-2α-propyl-6β-heptyl-trans-dekalin und
6 % trans,trans,4-Propylcyclohexylcyclohexan-4'-carbonsäure-trans-(4-propylcyclohexylester).

Beispiel H

Man stellt ein Gemisch her aus

12 % 2β-Cyan-2α-pentyl-6β-propyl-trans-dekalin
24 % 2β-Cyan-2α-heptyl-6β-propyl-trans-dekalin
21 % 2β-Cyan-2α-propyl-6β-pentyl-trans-dekalin
21 % 2β-Cyan-2α,6β-dipentyl-trans-dekalin
13 % 2β-Cyan-2α-propyl-6β-heptyl-trans-dekalin und
9 % trans-4-Pentylcyclohexancarbonsäure-(p-trans-4-butylcyclohexylphenylester).

Beispiel I

Man stellt ein Gemisch her aus

19 % 2β-Cyan-2α-heptyl-6β-propyl-trans-dekalin
18 % 2β-Cyan-2α,6β-dipentyl-trans-dekalin
11 % trans-4-Propylcyclohexancarbonsäure-(p-ethoxyphenylester)
9 % 4-(trans-4-Pentylcyclohexyl)-4'-(trans-4-propylcyclohexyl)-biphenyl
28 % 4-Ethyl-2'-fluor-4'-(trans-4-pentylcyclohexyl)-biphenyl und
15 % trans-1-(p-Ethoxyphenyl)-4-propylcyclohexan.

Beispiel J

Man stellt ein Gemisch her aus

18 % 2β-Cyan-2α-heptyl-6β-propyl-trans-dekalin
17 % 2β-Cyan-2α-propyl-6β-pentyl-trans-dekalin
17 % 2β-Cyan-2α,6β-dipentyl-trans-dekalin
10 % 2β-Cyan-2α-propyl-6β-heptyl-trans-dekalin
24 % p-(trans-4-Propylcyclohexyl)-benzoesäure-(4-butyl-2-cyanphenylester) und
14 % trans-1-(p-Ethoxyphenyl)-4-propylcyclohexan.

Beispiel K

Man stellt ein Gemisch her aus

19 % 2β-Cyan-2α-pentyl-6β-propyl-trans-dekalin
31 % 2β-Cyan-2α-propyl-6β-pentyl-trans-dekalin
33 % 2β-Cyan-2α,6β-dipentyl-trans-dekalin und
17 % 4-(trans-4-Pentylcyclohexyl)-4'-(trans-4-propylcyclohexyl)-biphenyl.

Beispiel L

Man stellt ein Gemisch her aus

11 % 2β-Cyan-2α-pentyl-6β-propyl-trans-dekalin
25 % 2β-Cyan-2α-heptyl-6β-propyl-trans-dekalin
21 % 2β-Cyan-2α-propyl-6β-pentyl-trans-dekalin
22 % 2β-Cyan-2α,6β-dipentyl-trans-dekalin
13 % 2β-Cyan-2α-propyl-6β-heptyl-trans-dekalin und
8 % p-(trans-4-Propylcyclohexyl)-benzoesäure-(trans-4-propylcyclohexylester).

Beispiel M

Man stellt ein Gemisch her aus

22 % 2β-Cyan-2α-heptyl-6β-propyl-trans-dekalin
20 % 2β-Cyan-2α,6β-dipentyl-trans-dekalin
18 % trans-4-Pentylcyclohexancarbonsäure-(trans-4-propylcyclohexylester)
10 % 4-(trans-4-Pentylcyclohexyl)-4'-(trans-4-propylcyclohexyl)-biphenyl und
30 % 4-Ethyl-2'-fluor-4'-(trans-4-pentylcyclohexyl)-biphenyl.

Beispiel N

Man stellt ein Gemisch her aus

17 % 2β-Cyan-2α,6β-dipentyl-trans-dekalin
43 % trans-4-Propylcyclohexancarbonsäure-(trans-4-propylcyclohexylester)
16 % trans-4-Pentylcyclohexancarbonsäure-(trans-4-propylcyclohexylester) und
17 % p-(trans-4-Propylcyclohexyl)-benzoesäure-(4-butyl-2-cyanphenylester).

**Patentansprüche**

1. Trans-Dekalincarbonitrile der Formel I

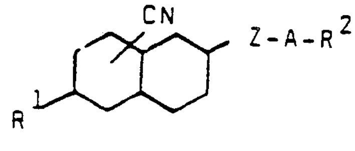

(I)

worin

$R^1$ eine Alkylgruppe mit 1-10 C-Atomen, worin bis zu zwei nicht benachbarte $CH_2$-Gruppen durch O-Atome ersetzt sein können,

Z $-CH_2CH_2-$, $-CO-O-$, $-O-CO-$, $-O-CH_2-$, $-CH_2O-$ oder eine Einfachbindung,

A 1,4-Phenylen, 1,4-Cyclohexylen, 1,3-Dioxan-2,5-diyl, 1,4-Bicyclo(2,2,2)-octylen, Pyrimidin-2,5-diyl oder eine Einfachbindung,

$R^2$ eine Alkylgruppe mit 1 bis 10 C-Atomen, worin bis zu zwei nicht benachbarte $CH_2$-Gruppen durch O-Atome ersetzt sein können, F, Cl, Br, CN, —O—$COR^2$ oder —$COOR^3$, oder, falls mindestens eine der Gruppen Z und A nicht für eine Einfachbindung steht, auch H, und

$R^3$ Alkyl mit 1 bis 6 C-Atomen

bedeuten, sowie die Säureadditionssalze der basischen unter diesen Verbindungen.

2. Verfahren zur Herstellung von trans-Dekalincarbonitrilen der Formel I nach Anspruch 1, dadurch gekennzeichnet, daß man eine Verbindung, die sonst der Formel I entspricht, aber an Stelle von H-Atomen eine oder mehrere reduzierbare Gruppen und/oder C—C-Bindungen enthält, mit einem reduzierenden Mittel behandelt,
oder daß man an eine Verbindung, die sonst der Formel I entspricht, aber im Dekalinrest keine CN-Gruppe, aber eine zusätzliche Doppelbindung enthält, HCN anlagert,
oder daß man zur Herstellung von Estern der Formel I (worin Z —CO—O— oder —O—CO— oder worin $R^2$ —O—$COR^3$ oder —$COOR^3$ bedeutet) eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von Dioxanderivaten der Formel I (worin A 1,3-Dioxan-2,5-diyl bedeutet) einen entsprechenden Aldehyd mit einem entsprechenden Diol umsetzt,
oder daß man ein entsprechendes Carbonsäureamid dehydratisiert oder ein entsprechendes Carbonsäurehalogenid mit Sulfamid umsetzt oder eine entsprechende Chlor- oder Bromverbindung mit einem Cyanid umsetzt,
oder daß man zur Herstellung von Dekalin-2- oder -6-carbonitrilen der Formel I ein Nitril der Formel II

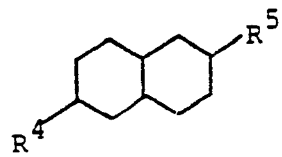

(II)

worin
(a) $R^4$ CN und $R^5$ —Z—A—$R^2$ oder
(b) $R^4$ $R^1$ und $R^5$ CN
bedeuten und
$R^1$, A, Z und $R^2$ die angegebenen Bedeutungen haben
mit einer Verbindung der Formel III

Q—X III

worin
Q (a) $R^1$ oder (b) $R^2$—A—Z— und
X Cl, Br, J, OH oder eine reaktionsfähig veresterte OH-Gruppe
bedeuten und
$R^1$,A, Z und $R^2$ die angegebenen Bedeutungen haben, umsetzt,
oder daß man eine Verbindung, die der Formel I entspricht, aber an Stelle einer C-C-Bindung zwischen dem die CN-Gruppe tragenden C-Atom und einem diesem benachbarten C-Atom ein zusätzliches H-Atom (an dem die CN-Gruppe tragenden C-Atom) und eine zusätzliche Gruppe X (an dem diesem benachbarten C-Atom, wobei X die angegebene Bedeutung hat) enthält, unter Abspaltung von HX cyclisiert,
oder daß man zur Herstellung von Ethern der Formel I (worin $R^1$ und/oder $R^2$ Alkylketten bedeuten worin bis zu 2$CH_2$-Gruppen durch O-Atome ersetzt sind und/oder Z eine —$OCH_2$- oder —$CH_2O$-Gruppe ist) eine entsprechende Hydroxyverbindung verethert,
und/oder daß man gegebenenfalls eine Base der Formel I durch Behandeln mit einer Säure in eines ihrer Säureadditionssalze umwandelt,
oder daß man gegebenenfalls eine Verbindung der Formel I aus einem ihrer Säureadditionssalze durch Behandeln mit einer Base freisetzt.

3. Verwendung der Verbindungen der Formel I nach Anspruch 1 als Komponenten flüssigkristalliner Dielektrika für elektrooptische Anzeigeelemente.

4. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

5. Elektrooptisches Anzeigeelement, dadurch gekennzeichnet, daß es ein Dielektrikum nach Anspruch 4 enthält.

**Claims**

1. Trans-Decalin-carbonitriles of the formula I

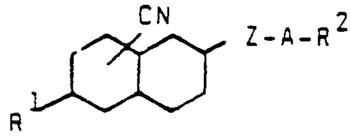

(I)

wherein

$R^1$ is an alkyl group with 1-10 C atoms, it being possible for up to two non-adjacent $CH_2$ groups to be replaced by O atoms,

Z is $-CH_2CH_2-$, $-CO-O-$, $-O-CO-$, $-O-CH_2-$, $-CH_2O-$ or a single bond,

A is 1,4-phenylene, 1,4-cyclohexylene, 1,3-dioxane-2,5-diyl, 1,4-bicyclo(2,2,2)-octylene, pyrimidine-2,5-diyl or a single bond, and

$R^2$ is an alkyl group with 1 to 10 C atoms, it being possible for up to two non-adjacent $CH_2$ groups to be replaced by O atoms, or is F, Cl, Br, CN, $-O-COR^3$ or $-COOR^3$, or, if at least one of the groups Z and A is not a single bond, can also be H and

$R^3$ is alkyl of 1 to 6 C atoms,

and the acid addition salts of basic compounds of this type.

2. Process for the preparation of trans-decalin-carbonitriles of the formula I according to Claim 1, characterised in that a compound which otherwise corresponds to the formula I but contains one or more reducible groups and/or C-C bonds instead of H atoms is treated with a reducing agent, or in that HCN is added onto a compound which otherwise corresponds to the formula I but contains no CN group in the decalin radical but an additional double bond,

or in that, for the preparation of esters of the formula I (wherein Z is $-CO-O-$ or $-O-CO-$, or wherein $R^2$ is $-O-COR^3$ or $-COOR^3$), a corresponding carboxylic acid or one of its reactive derivatives is reacted with a corresponding alcohol or one of its reactive derivatives,

or in that, for the preparation of dioxane derivatives of the formula I (wherein A is 1,3-dioxane-2,5-diyl), a corresponding aldehyde is reacted with a corresponding diol,

or in that a corresponding carboxylic acid amide is dehydrated or a corresponding carboxylic acide halide is reacted with sulfamide

or a corresponding chlorine or bromine compound is reacted with a cyanide,

or in that, for the preparation of decalin-2- or -6-carbonitriles of the formula I, a nitrile of the formula II

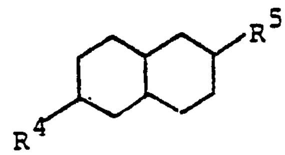

(II)

wherein

(a) $R^4$ is CN and $R^5$ is $-Z-A-R^2$, or

(b) $R^4$ is $R^1$ and $R^5$ is CN, and

$R^1$, A, Z and $R^2$ have the meanings given, is reacted with a compound of the formula III

$$Q-X \qquad III$$

wherein

Q is (a) $R^1$ or (b) $R^2-A-Z-$ and

X is Cl, Br, I, OH or a reactive esterified OH group, and

$R^1$, A, Z and $R^2$ have the meanings given,

or in that a compound which corresponds to the formula I but, instead of a C-C bond between the C atom carrying the CN group and a C atom adjacent to this, contains an additional H atom (on the C atom carrying the CN group) and an additional group X (on the C atom adjacent to this, X having the meaning given), is cyclised, HX being split off,

or in that, for the preparation of ethers of the formula I (wherein $R^1$ and/or $R^2$ are alkyl chains, up to 2 $CH_2$ groups being replaced by O atoms, and/or Z is a $-OCH_2-$ or $-CH_2O-$ group), a corresponding hydroxy compound is etherified,

and/or in that, if appropriate, a base of the formula I is converted into one of its acid addition salts by treatment with an acid,

or in that, if appropriate, a compound of the formula I is liberated from one of its acid addition salts by treatment with a base.

3. Use of the compounds of the formula I according to Claim 1 as components of liquid crystal dielectrics for electrooptical display elements.

4. Liquid crystal dielectric for electrooptical display elements with at least two liquid crystal components, characterised in that at least one component is a compound of the formula I according to

Claim 1.

5. Electrooptical display element, characterised in that it contains a dielectric according to Claim 4.

**Revendications**

1. Trans-décalinecarbonitriles de formule

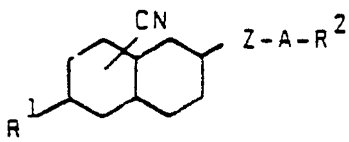

(I)

dans laquelle

$R^1$ représente un groupe alcoyle ayant 1 à 10 atomes de carbone, jusqu'à deux groupes $CH_2$ non vicinaux pouvant être remplacés par des atomes O,

Z $-CH_2CH_2-$, $-CO-O-$, $-O-CO-$, $-O-CH_2-$, $-CH_2O-$ ou une simple liaison,

A 1,4-phénylène, 1,4-cyclohexylène, 1,3-dioxanne-2,5-diyle, 1,4-bicyclo(2,2,2)-octylène, pyrimidine-2,5-diyle ou une simple liaison,

$R^2$ un groupe alcoyle ayant 1 à 10 atomes de carbone, jusqu'à deux groupes $CH_2$ non vicinaux pouvant être remplacés par des atomes O ; ou bien F, Cl, Br, CN, $-O-COR^3$ ou $-COOR^3$, ou bien, au cas où au moins un des groupes Z et A n'est pas une simple liaison, également H, et

$R^3$ un alcoyle ayant 1 à 6 atomes de carbone,

de même que les sels d'addition d'acides des composés basiques parmi ces composés.

2. Procédé de préparation de trans-décalinecarbonitriles de formule I selon la revendication 1, caractérisé en ce qu'on traite un composé, qui à part cela répond à la formule I mais qui au lieu des atomes H contient un ou plusieurs groupes réductibles et/ou des liaisons C-C, avec un agent réducteur, ou bien en ce qu'on fixe du HCN sur un composé qui, à part cela, répond à la formule I mais ne contient pas dans le radical décaline un groupe CN mais contient une double liaison supplémentaire,

ou bien en ce que pour la préparation d'esters de formule I (dans laquelle Z signifie $-CO-O-$ ou $-O-CO-$ ou dans laquelle $R^2$ signifie $-O-COR^3$ ou $-COOR^3$), on fait réagir un acide carboxylique correspondant ou un de ses dérivés réactifs avec un alcool correspondant ou un de ses dérivés réactifs,

ou bien en vue de la préparation de dérivés de dioxanne de formule I (où A signifie 1,3-dioxanne-2,5-diyle), on fait réagir une aldéhyde correspondante avec un diol correspondant,

ou bien en ce qu'on déshydrate une carboxamide correspondante ou bien on fait réagir un halogénure d'acide carboxylique correspondant avec de la sulfamide,

ou bien un composé chloré ou bromé avec un cyanure,

ou bien, pour la préparation de décaline-2- ou -6- carbonitriles de formule I, en ce qu'on fait réagir un nitrile de formule II :

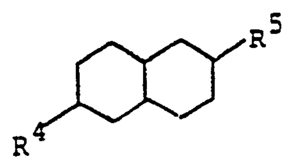

(II)

dans laquelle

(a) $R^4$ signifie CN et $R^5$ $-Z-A-R^2$ ou

(b) $R^4$ signifie $R^1$, et $R^5$ signifie CN

et

$R^1$, A, Z et $R^2$ ont les significations indiquées,

avec un composé de formule III

$$Q-X \qquad III$$

dans laquelle

Q signifie (a) $R^1$ ou (b) $R^2-A-Z-$ et

X Cl, Br, I, OH ou un groupe OH estérifié réactif

et

$R^1$, A, Z et $R^2$ ont les significations indiquées,

ou bien en ce qu'on cyclise, avec clivage de HX, un composé qui répond à la formule I mais qui contient au lieu d'une liaison C-C entre l'atome de carbone porteur du groupe CN et un atome de carbone voisin de celui-ci un atome H supplémentaire (sur l'atome de carbone porteur du groupe CN) et un groupe X supplémentaire (sur l'atome de carbone voisin de celui-ci, X ayant la signification indiquée),

ou en ce que pour la préparation d'éthers de formule I (où $R^1$ et/ou $R^2$ signifient des chaînes alcoyle, cas où jusqu'à 2 groupes $CH_2$ sont remplacés par des atomes O et/ou Z est un groupe $-OCH_2-$ ou $-CH_2O-$), on éthérifie un composé hydroxylé correspondant,
et/ou en ce qu'on convertit éventuellement une base de formule I par traitement avec un acide en un de ses sels d'addition d'acides,
ou en ce qu'éventuellement on met en liberté un composé de formule I à partir d'un de ses sels d'addition d'acides par traitement avec une base.

3. Utilisation des composés de formule I selon la revendication 1 comme composants diélectriques à cristaux liquides pour éléments indicateurs électro-optiques.

4. Diélectrique à cristaux liquides pour éléments indicateurs électro-optiques avec au moins deux composants à cristaux liquides, caractérisé en ce qu'au moins un composant est un composé de formule I selon la revendication 1.

5. Elément indicateur électro-optique, caractérisé en ce qu'il contient un diélectrique selon la revendication 4.